# EUROPEAN PATENT SPECIFICATION

(11) **EP 3 331 486 B1**
(45) Date of publication and mention of the grant of the patent: **07.10.2020**
(21) Application number: 16779172.2
(22) Date of filing: 02.08.2016
(51) Int. Cl.: A61H 21/00, A61B 1/00, A61B 1/04

(54) **APPARATUS FOR TREATING CONSTIPATION AND REMOVING FECALOMAS**
VORRICHTUNG ZUR BEHANDLUNG VON VERSTOPFUNG UND ENTFERNUNG VON FEKALOMEN
APPAREIL DE TRAITEMENT DE CONSTIPATION ET DE RETRAIT DE FÉCALOMES

(30) Priority: 06.08.2015 IT UB20152953
(43) Date of publication of application: 13.06.2018
(73) Proprietor: Sabatini, Tony, 25126 Brescia (BR) (IT)
(72) Inventor: Sabatini, Tony, 25126 Brescia (BR) (IT)
(74) Representative: Maroscia, Antonio
(86) International application number: PCT/IB2016/054636
(87) International publication number: WO 2017/021876

(56) References cited:
- WO-A2-02/082979
- WO-A2-03/005877
- WO-A2-2009/060460
- DE-A1-102011 089 334
- US-A1- 2007 238 940
- WERNER WEITSCHIES ET AL: "High-Resolution Monitoring of the Gastrointestinal Transit of a Magnetically Marked Capsule", JOURNAL OF PHARMACEUTICAL SCIENES, vol. 86, no. 11, 1 November 1997 (1997-11-01), pages 1218-1222, XP009192392,

## Description

### Field of the invention

The present invention generally finds application in the field of medical apparatus for treating gastrointestinal disorders and particularly relates to an apparatus for treating constipation, more particularly in cases of slower bowel movement or coprostasis, fecal impaction or formation of fecalomas.

### Background Art

Serious constipation is known to be treated using drugs for stimulating intestinal wall muscles to facilitate expulsion of its contents, such as laxatives.

Laxatives may be classified in various groups, including hydrophilic or mass laxatives, which increase gut contents to stimulate physiological intestinal motility, i.e. peristalsis, emollient laxatives which only have a lubricating action, osmotic laxatives, composed of non-reabsorbable substances which retain water in the gut, thereby increasing the volume of its contents and making them softer to stimulate spontaneous peristalsis, and finally synthetic or natural contact laxatives, whose action consists in stimulating intestinal wall muscles to facilitate expulsion of its contents.

Furthermore, drugs are currently available, which act on the nerve plexuses of intestinal muscles and are known as prokinetic drugs, but their effectiveness is often temporary or limited to the administration route, e.g. intramuscular, subcutaneous or intravenous route, or by side effects.

Finally, clysmas and clysters are available for anal treatment of constipation and removal of fecalomas, by hydration of fecalomas, but are often ineffective especially when the fecaloma is situated in the right colon or in case of compromised peristalsis.

A first drawback of these drugs is that they can irritate the intestinal wall, cause habituation, produce pain or offset the osmotic balance of the gut.

A further drawback is that these drugs have reduced selectivity toward the different anatomical parts of the intestine, or a limited effective range.

These drawbacks generally lead to reduced effectiveness of these drugs in constipation treatment, as well as the occurrence of possible undesired side effects.

Furthermore, the above described drugs are particularly ineffective in case of paralytic ileus or colon pseudoobstruction, which causes stagnation of feces in the intestinal tract due to the lack of movement of the intestinal wall.

In an attempt to at least partially obviate these drawbacks, medical devices have been developed which exert a mechanical action on fecalomas to break them down for expulsion.

US2007/0238940 A1 discloses a magnetic gastrointestinal capsule designed to be ingested by the patient for breaking down fecalomas and moving the intestinal contents by means of vibrations.

The capsule has an outer enclosure which is designed to contain a vibrating system comprising an axial thrust-imparting device which acts on the enclosure. The thrust-imparting device is connected to an electric motor and an electronic board which is programmable to promote the vibrating motion in the gut. The capsule is moved forward in the gut by peristalsis of the smooth wall thereof and later starts to vibrate with a predetermined delay that can be set in the electronic control board.

A first drawback of this prior art arrangement is its considerable complexity and, as a result, its high cost.

A further drawback is that the capsule is positioned in the gut by a highly complex and inaccurate procedure, which solely depends on the peristalsis of the patient's gut.

Due to these positioning difficulties, the capsule might start to vibrate before reaching the intestinal site containing fecalomas, thereby actually having no therapeutic effect.

A further drawback is that the parts of the vibrating system are exposed to wear and require constant maintenance to prevent malfunctioning.

Another drawback is that the parts that are used to form the capsule have low compatibility with the organism and might cause inflammation or infection in the intestinal wall.

Finally, another drawback is that the capsule cannot ensure transport of fecaloma fragments once a fecaloma has been broken down, such drawback being particularly felt in case of slow peristalsis of the colon wall.

### Technical Problem

In light of the above discussed prior art, the technical problem to be solved consists in providing an apparatus that is as simple as possible and can mechanically break down fecalomas, and transport and remove their fragments, by facilitating control of their position and movement in the gut, particularly in case of coprostasis causing fecal impaction and formation of fecalomas.

### Disclosure of the invention

The object of the present invention is to solve the above mentioned technical problem and obviate the above discussed drawbacks, by providing an apparatus for treatment of constipation and removing fecalomas that is highly efficient and relatively cost-effective.

A particular object of the present invention is to provide an apparatus for treatment of constipation as discussed above that can ensure quick and accurate positioning of a device for mechanically breaking down fecalomas.

Another object of the present invention is to provide an apparatus for treatment of constipation as discussed above that can have an optimal operation even in case of inefficient intestinal motility.

A further object of the present invention is to provide an apparatus for treatment of constipation as discussed above that can accurately and repeatably control the movement of the ingestible element for breaking down fecalomas.

A particular object of the present invention is to provide an apparatus for treatment of constipation as discussed above that has a simple and easily formed construction.

Another object of the present invention is to provide an apparatus for treatment of constipation as discussed above that can be easily used even by not specially skilled health care operators.

A further object of the present invention is to provide an apparatus for treatment of constipation as discussed above that reduces the risk of inflammation or irritation of the intestinal wall.

These and other objects, as better explained hereafter, are fulfilled by an apparatus for treating constipation and removing impacted feces (coprostasis) and fecalomas as defined in claim 1.

With this configuration, the apparatus can mechanically transport the intestinal contents and the fecalomas by breaking down the latter and entrain them in the oral-aboral direction irrespective of the movement of the inner intestinal wall, which is required by other methods that always require intestinal peristalsis.

Advantageous embodiments of the invention are as defined in the dependent claims.

### Brief description of the drawings

Further features and advantages of the invention will be more readily apparent upon reading of the detailed description of a preferred non-exclusive embodiment of an apparatus for treatment of constipation, particularly in case of slow bowel movement (coprostasis) or formation of fecalomas according to the invention, which is illustrated by way of example and without limitation with the help of the following drawings, in which:
FIGG. 1A and 1B are lateral diagrammatic views of the apparatus of the invention according to two different embodiments, acting on a patient that suffers from constipation, represented in a sagittal section;
FIG. 2 is a diagrammatic front view of the apparatus of Fig. 1, acting on a patient that suffers from constipation, represented in a coronal section;
FIG. 3 is a simplified diagrammatic view with an enlargement of a first embodiment of a detail of the apparatus of Fig. 1;
FIG. 4 is a simplified diagrammatic view of a second embodiment of a detail of the apparatus of Fig. 1;
FIG. 5 is a simplified diagrammatic view of the apparatus for treating intestinal constipation according to a second embodiment;
FIGS. 6 to 10 are diagrammatic views of the operating steps of application of the apparatus of the invention.

### Detailed description of a preferred exemplary embodiment

The figures particularly show an apparatus for treating constipation, particularly in case of slow bowel movement, or coprostasis, which causes fecal impaction and formation of fecalomas F, according to the invention, generally designated by numeral 1.

The figures show diagrammatic sagittal and coronal sections of a patient P that has fecalomas F or impacted feces in the colon C.

The apparatus 1 is designed to mechanically interact with the intestinal contents, particularly in case of impacted feces due to coprostasis or fecalomas F that form at the colon C, thereby avoiding the use of pharmaceuticals that are often ineffective and may have significant side effects for the patient P.

Advantageously, the apparatus 1 may be also suitable for veterinary use, particularly with mammals or pets predisposed to fecaloma formation level with the colon, with the same operating principles as used for humans.

In its basic form, the apparatus 1 comprises an element 2 that can be ingested by a patient P and contains a base material having ferromagnetic properties.

Advantageously, the ingestible element 2 is insoluble, non-absorbable and capable of withstanding the gastrointestinal environment to reach the colon C of the patient P in an unaltered state and be expelled with the feces.

Multiple ingestible elements 2 may be possibly provided, which are adapted to exert a simultaneous mechanical action to break down, removal and transport of the fecaloma F in the oral-aboral direction.

The apparatus 1 further comprises a magnetic device 3 situated outside the body B of the patient P and adapted to interact with the ingestible element 2, particularly when the latter is in the colon C.

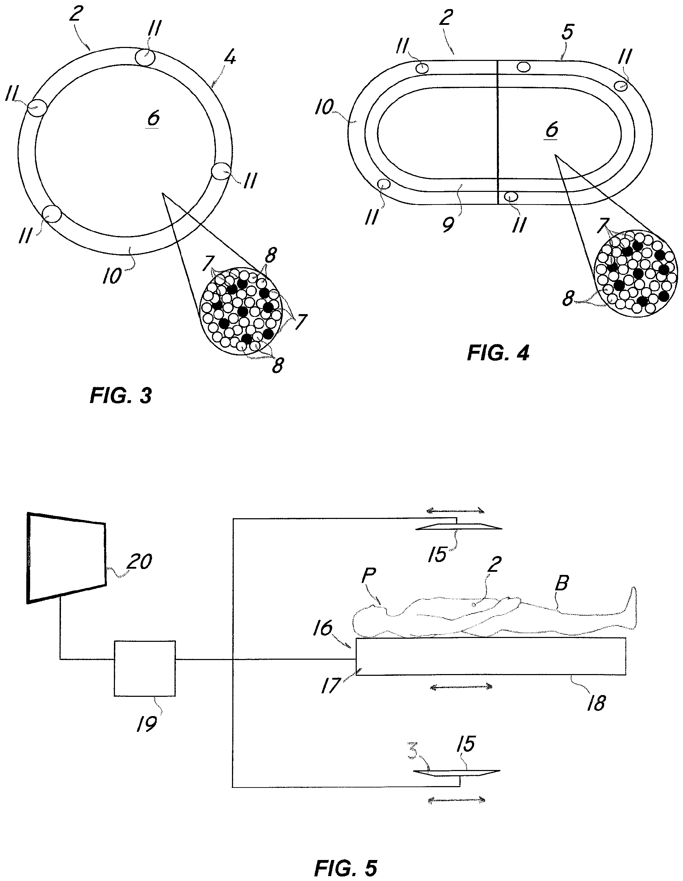

As diagrammatically shown in FIGS. 3 and 4, the ingestible element may consist of a tablet 4 or a capsule 5 and may have a round, spherical, oblong or ovoid shape, which will facilitate not only its ingestion through the mouth, but also its forward movement through the esophagus, the duodenum, the stomach, the small intestine and the large intestine, to the colon C.

Conveniently, the ingestible element 2 may have a central core 6 containing a core material, which may in turn comprise at least one ferromagnetic compound 7 in the form of a powder dispersed in chemically inert matrix particles 8.

Preferably, the ferromagnetic compound 7 will be selected from the group comprising magnetite, whereas the chemically inert matrix 8 may be selected from the group comprising hydroxypropyl methylcellulose.

The inert matrix 8 may further comprise appropriate glidants, thinners and lubricants dispersed among the particles of core material, to impart a more regular shape thereto and reduce the friction typically occurring among them.

The particles of the inert matrix 8 and the ferromagnetic compound 7 will be blended before the encapsulation or compression steps and may be substantially 70 and 30% by weight, as schematically shown in the enlarged part of FIG. 3.

Nevertheless, these amounts are given by way of example and without limitation, and may be also different and have different compositions, with the inert matrix component 8 being always predominant relative to the ferromagnetic compound 7.

Thus, the particles of the ferromagnetic compound 7 will be diluted and uniformly dispersed in the inert matrix 8, which will further increase their biocompatibility with respect to the gastrointestinal tract environment.

In the embodiment with a capsule 5 as an ingestible element 2, the central core 6 will be enclosed in an outer protective layer 9 comprising gelatin, as is known per se, and may have a volume ranging from 0,5 to 5 ml.

In the embodiment with a tablet 4 as an ingestible element 2, the latter may have a weight ranging from 300 to 1000 mg and a diameter ranging from 5 mm to 20 mm.

Furthermore, the power of the magnetic device 3 may be also changed according to the size of the individual or, for veterinary use, of the animal. The intensity of the magnetic field, and hence the size and/or the power of the magnetic device 3, will depend on the age of the patient and on other anatomical factors, such as body weight, abdominal volume and conformation.

Thus, the tablet 4 will be very easily ingested and will be relatively non-invasive as it passes through the gastrointestinal tract of the patient P.

Preferably, the central core 6 may be covered by a biocompatible outer layer 10 which is adapted to prevent direct exposure thereof to the body B of the patient P, as best shown in FIGS. 3 and 4.

Particularly, the outer layer 10 directly contacts the protective layer 9 of the capsule 5, as best shown in FIG. 4.

The layer 10 may be made of a material selected from the group comprising polymers with free functional groups such as dextran, polyethylene glycol (PEG), polyethylene oxide, or alternatively it may comprise gold, carbon or silica.

Advantageously, the layer 10 may comprise an active ingredient 11 that can be released into the colon C and dispersed therein, as shown in FIGS. 3 and 4, or is alternatively chemically bonded to the free functional groups of the polymeric material.

This active ingredient 11 may comprise drugs selected from the group comprising laxatives or milk enzymes.

The magnetic device 3 can be placed outside the body B of the patient P level with the colon C and is adapted to promote controlled movement of the ingestible element 2 in the colon C, breakdown and transport of fecalomas F in the aboral direction, even in case of disturbed peristalsis in the patient.

Such movement may be repeated to cause mechanical breakdown of the fecaloma F due to impacts between the latter and the ingestible element 2, and hence removal of the obstruction at the colon C.

The apparatus 1 of the invention has a small number of parts and is very simple, although it allows accurate control of the movements of the ingestible element 2 for breaking down the fecaloma F.

Preferably, the magnetic device 3 is adapted to promote controlled movement of the ingestible element 2 level with the ascending colon C of the patient P.

This is because, due to its particular anatomical conformation, this region of the gastrointestinal tract is most exposed to the formation of fecalomas F which hinder normal intestinal function.

Advantageously, the controlled movement of the ingestible element 2 in the colon C for breaking down and pushing the fecalomas F is obtained by appropriate movement means 12 associated with the magnetic device 3.

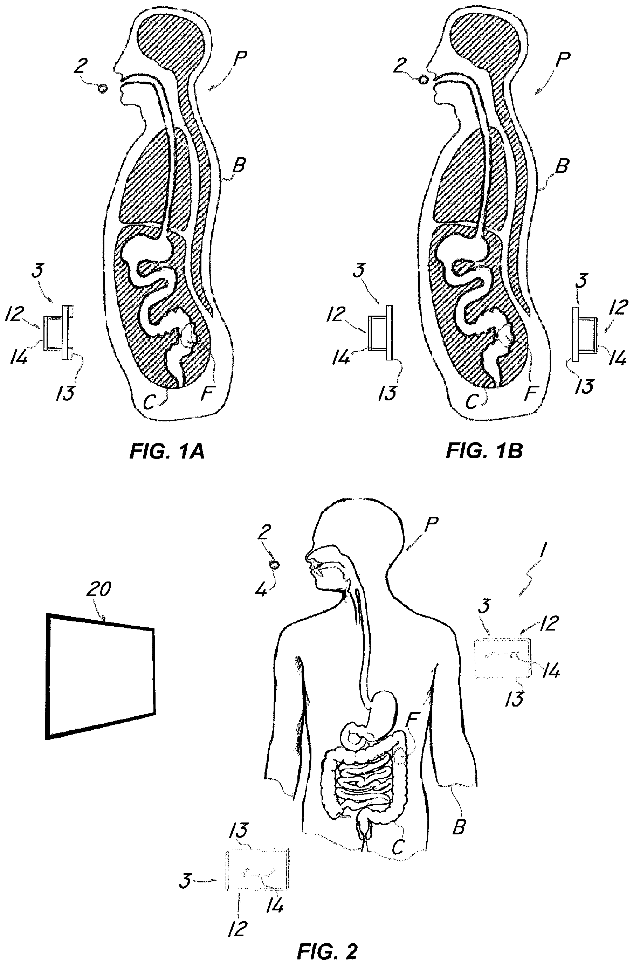

In the embodiment of the invention as shown in FIG. 1A, the motion-imparting means 12 of the magnetic device 3 comprise at least one external permanent magnet 13, e.g. an annular shape associated with a respective support 14 that is adapted to be held be an operator to apply a magnetic field to the region of the colon C and defining movement means 12.

The permanent magnet 13 may be located in a ventral-anterior position relative to the patient P and will be moved by the operator as described above. The use of a single permanent magnet 13 is preferred for patients having a small body surface or a low body mass index and a flat belly.

In the preferred embodiment of the invention, as best shown in FIG. 1B, the magnetic device 3 comprises a pair of permanent magnets 13 with respective supports 14 designed to be held by an operator. The permanent magnets 13 may be located in a ventral-anterior and dorsal-posterior position relative to the patient.

Conveniently, the permanent magnets 13 may comprise a pair of plates of appropriate size, such that the region of the body B of the patient P corresponding to the colon C is entirely covered.

In an alternative embodiments, as best shown in FIG. 5 and particularly suitable for elder, handicapped, sedated or bedridden patients, the magnetic device 3 may comprise at least one pair of electromagnets 15 placed on opposite sides relative to the patient P and the apparatus 1 comprises a support 16 for the body of the patient P.

Here, the movement means 12 consist of the support 16 that will be moved to impart the movement relative to the ingestible element 2.

Particularly, the moving support 16 may comprise a bed 17 with a base18 that can be horizontally translated to change its position relative to the pair of electromagnets 5.

In this embodiment, both the moving support 16 and the electromagnets 15 may be connected to an electronic control unit 19 for selective power supply and adjustment of the position of the supports 16 and the electromagnets 15.

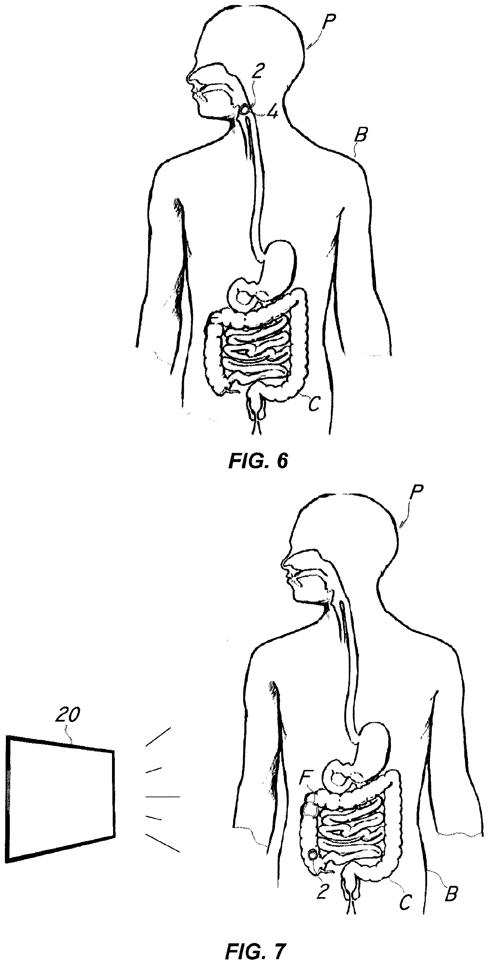

The apparatus 1 further comprises, as best shown in FIGS. 2 and 7, image-based diagnostic means 20 for detecting the position of the ingestible element 2 in the colon C before and after movement thereof by the magnetic device 3.

Advantageously, the diagnostic means 20 may comprise a normal X-ray imaging apparatus, which can image the active element 2 due to its radiopacity, or a suitable magnetic instrument and interface means, not shown, which can be consulted by the operator to check the exact position of the ingestible element 2 in the colon C of the patient.

Furthermore, in the embodiment as shown in FIG. 5, the diagnostic means 20 may be also connected to the control unit 19 as described above.

This step may be preceded by a diagnostic determination of the nature and conformation of the fecaloma F and the health state of the patient P, for the operator to select the most appropriate treatment.

Then, once a predetermined time has been allowed to elapsed, for the ingestible element 2 to reach the ascending colon C, the patient P undergoes a diagnostic examination using the above discussed diagnostic means 20 to detect the exact position of the ingestible element 2, as shown in FIG. 7.

In case of a negative determination an additional predetermined time is allowed to elapse for the ingestible element 2 to reach the ascending colon C.

In case of a positive determination, one or both of the external magnets 13 are placed at the colon C of the patient P, in front of and behind the body B thereof respectively.

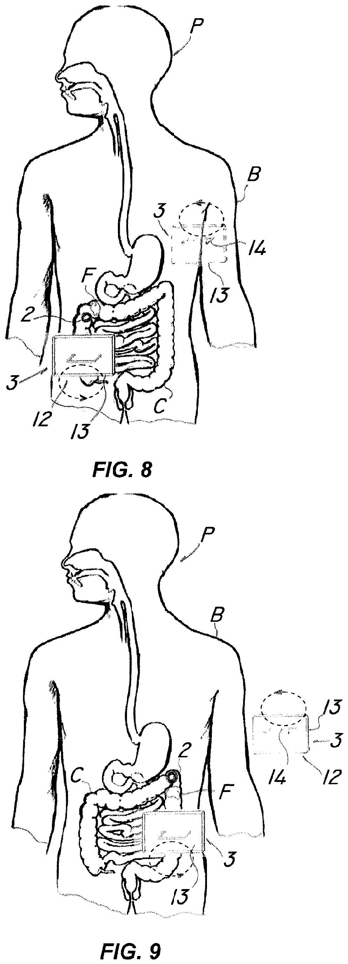

Then, as shown in FIGS. 8 and 9, the external magnets 13 are transversely moved by rotary peripheral movements to cause the ingestible element 2 to be displaced in the colon C of the patient P.

If the tablet 4 or capsule 5 have a shape other than the spherical shape, the movement of the magnets 13 can change their orientation relative to the fecaloma F.

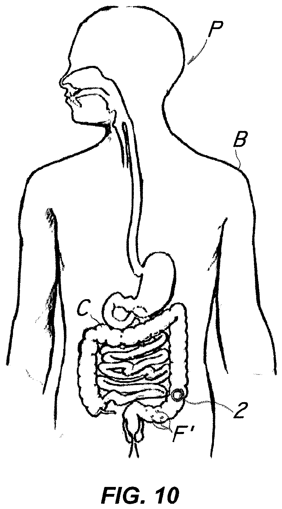

Such displacement causes the ingestible element 2 to hit against the walls of the fecaloma F and is repeated until the latter is completely broken down, as shown in FIG. 10.

Advantageously, the magnets 13 may be placed at the sides of the body B of the patient P as they move to allow controlled and accurate displacement of the ingestible element 2 in the colon C.

In addition to breaking down the fecaloma F, the displacement of the ingestible element 2 in the colon C also causes the movement of its fragments F' along the other sections of the colon C.

Particularly, these movements allow the ingestible element 2 to be displaced from the right iliac fossa below which the first section of the ascending colon, i.e. the blind intestine, is usually situated, first toward the right hypochondrium, as shown in FIG. 8, where the ascending colon ends and the transverse colon starts with the hepatic flexure.

Then, the ingestible element 2 is displaced toward the left hypochondrium, as shown in FIG. 9, along the entire transverse colon to the splenic flexure of the colon and finally toward the left iliac fossa, along the left colon, to the rectum and the anus.

Then, the fragments F' of the fecaloma F and the ingestible element 2 are expelled from the body B of the patient P by normal defecation, as shown in FIG. 10.

If the apparatus 1 includes the pair of electromagnets 15, after diagnostic examination the patient P is laid in a supine position on the bed 17 and the latter or the electromagnets 15 are displaced to adjust the attraction force acting on the ingestible element 2 and its position in the colon C.

Furthermore, the apparatus may be also considered to be used in patients with abdominal electronic devices, such as stimulators or pacemakers, once the applicability and compatibility of the magnetic field to and with these devices has been confirmed.

The apparatus for treating fecalomas in constipation with impacted feces and fecalomas according to the invention is susceptible of a number of changes and variants within the inventive principle disclosed in the annexed claims.

All the details thereof may be replaced by other technically equivalent parts, and the materials may vary depending on different needs, without departure from the scope of the invention.

While the apparatus for treating constipation has been described with particular reference to the annexed figures, the numerals referred to in the disclosure and claims are only used for the sake of a better intelligibility of the invention and shall not be intended to limit the claimed scope in any manner.

### Industrial Applicability

The present invention can be used in industry, as it can be produced on an industrial scale by factories producing medical equipment for treating gastrointestinal disorders.

## Claims

1. An apparatus (1) for treating constipation and removing fecalomas (F), comprising:
- at least one element (2) designed to be ingested by a patient (P), which is in the form of a tablet (4) or capsule (5) and has ferromagnetic properties;
- a magnetic device (3) adapted to interact with said ingestible element (2) through the body (B) of the patient (P);
wherein said magnetic device (3) is designed to be placed outside the patient (P), in correspondence of his/her/its colon (C), movement means (12) being associated with said magnetic device (3) to promote controlled movement of said ingestible element (2) in the colon (C);
wherein said ingestible element (2) comprises an outer layer (10) that encloses a core (6) of a material comprising at least one ferromagnetic compound (7) in powder form dispersed in a chemically inert matrix (8), said outer layer (10) being biocompatible to avoid direct exposure of the core (6) to the body (B) of the patient (P);
**characterized in that** said magnetic device (3) comprises at least one permanent magnet (13) associated with a respective support (14) that is designed to be held by an operator and defines said movement means (12), said magnetic device (3) being adapted to be repeatedly moved to cause the mechanical breakdown of the fecalomas (F) due to impacts between the latter and the ingestible element (2) and the removal of the obstruction at the colon (C), said outer layer (10) being insoluble, non-absorbable and capable of withstanding the gastrointestinal environment, such that said ingestible element (2) may reach the colon (C) of the patient (P) in intact form, contact, breakdown and push the fecalomas (F) and cause the intestinal contents to move forward in the aboral direction and be expelled in an unaltered state with the feces, further comprising a support (16) for the body (B) of the patient (P) and **in that** said magnetic device (3) further comprises at least one pair of electromagnets (15) placed on opposite sides relative to the patient (P), said movement means (12) consisting of said support (16) for imparting the movement relative to said ingestible element (2).

2. An apparatus as claimed in claim 1, **characterized in that** said core (6) is enclosed in an outer protective layer (9) that is in turn covered by and in direct contact with said outer layer (10).

3. An apparatus as claimed in claim 1, **characterized in that** said chemically inert matrix (8) is selected from the group comprising hydroxypropyl methylcellulose.

4. An apparatus as claimed in claim 1, **characterized in that** said at least one ferromagnetic compound (7) is selected from the group comprising magnetite.

5. An apparatus as claimed in claim 1, **characterized in that** the particles of said chemically inert matrix (8) are blended with the ferromagnetic compound (7) before encapsulation or compression, the particles of the inert matrix (8) being predominant by weight relative to the particles of said ferromagnetic compound (7).

6. An apparatus as claimed in claim 5, **characterized in that** the amount of inert matrix (8) is 70% by weight and the amount of ferromagnetic compound (7) is 30% by weight.

7. An apparatus as claimed in claim 1, **characterized in that** said outer layer (10) is made of a material selected from the group comprising polymers with free functional groups such as dextran, polyethylene glycol (PEG), polyethylene oxide (PEO), gold, activated carbon and silica

8. An apparatus as claimed in claim 1, **characterized in that** said inert matrix (8) further comprises glidants, thinners and lubricants dispersed among the particles of said ferromagnetic compound (7), to impart a more regular shape thereto and reduce the friction among them.

9. An apparatus as claimed in claim 1, **characterized in that** said ingestible element (2) in capsule form (5) has such dimensions as to have a volume ranging from 0.7 ml to 5 ml, and in tablet form (4) has such dimensions as to have a diameter ranging from 5 mm to 20 mm, in view of optimizing entrainment and removal of fecalomas (F).

10. An apparatus as claimed in claim 1, **characterized in that** it comprises image-based diagnostic means (20) which are adapted to interact with said ingestible element (2) to detect its position in the colon (C) before or during movement by said magnetic device (3), said diagnostic means (20) being selected from the group comprising an X-ray image analyzer.

11. Apparatus as claimed in one or more of claims 1 to 10, for use in medicine.

12. Apparatus as claimed in one or more of claims 1 to 10, for use in veterinary medicine.

## Patentansprüche

1. Ausrüstung (1) zur Behandlung von Verstopfung und Entfernung von Fäkalomen (F), umfassend:
- mindestens ein Element (2), das zur Aufnahme durch einen Patienten (P) bestimmt ist und in Form einer Tablette (4) oder Kapsel (5) vorliegt und ferromagnetische Eigenschaften aufweist;
- eine Magnetvorrichtung (3), die angepasst ist, um mit dem einnehmbaren Element (2) durch den Körper (B) des Patienten (P) zu interagieren;
wobei die Magnetvorrichtung (3) so ausgelegt ist, dass sie außerhalb des Patienten (P) entsprechend seinem/ihrem Dickdarm (C) platziert werden kann, wobei Bewegungseinrichtung (12) mit der Magnetvorrichtung (3) verbunden ist, um eine kontrollierte Bewegung des eingenommenen Elements (2) im Dickdarm (C) zu fördern;
wobei das einnehmbare Element (2) eine äußere Schicht (10) umfasst, die einen Kern (6) eines Materials umschließt, das mindestens eine ferromagnetische Zusammensetzung (7) in Pulverform umfasst, die in einer chemisch inerten Matrix (8) dispergiert ist, wobei die äußere Schicht (10) biokompatibel ist, um eine direkte Exposition des Kerns (6) gegenüber dem Körper (B) des Patienten (P) zu vermeiden;
**dadurch gekennzeichnet, dass** die Magnetvorrichtung (3) ferner mindestens einen Permanentmagneten (13) umfasst, der einem jeweiligen Träger (14) zugeordnet ist, der zum Halten durch einen Bediener ausgelegt ist und die Bewegungseinrichtung (12) definiert, wobei die Magnetvorrichtung (3) angepasst ist, um wiederholt bewegt zu werden, um den mechanischen Abbau der Fäkalien (F) aufgrund von Stößen zwischen diesem und dem einnehmbaren Element (2) und der Entfernung der Obstruktion am Dickdarm (C) zu verursachen; wobei der äußeren Schicht (10) unlöslich, nicht resorbierbar und in der Lage ist, der Magen-Darm-Umgebung zu widerstehen, so dass das einnehmbare Element (2) in intakter Form den Dickdarm (C) des Patienten (P) erreichen kann, und die Fäkalien (F) berühren, abbauen und drücken kann und somit bewirken, dass sich der Darminhalt in aboraler Richtung vorwärts bewegt und in einem unveränderten Zustand mit dem Kot ausgestoßen wird; wobei die Ausrüstung ferner eine Stütze (16) für den Körper (B) des Patienten (P) umfasst und die Magnetvorrichtung (3) ferner mindestens ein Paar Elektromagnete (15) umfasst, die auf gegenüberliegenden Seiten relativ zum Patienten (P) angeordnet sind, die Bewegungseinrichtung (12) bestehend aus der Stütze (16) zum Vermitteln der Bewegung relativ zu dem einnehmbaren Element (2).

2. Ausrüstung nach Anspruch 1, **dadurch gekennzeichnet, dass** der Kern (6) in einer äußeren Schutzschicht (9) eingeschlossen ist, die wiederum von der äußeren Schicht (10) bedeckt ist und in direktem Kontakt mit dieser steht.

3. Ausrüstung nach Anspruch 1, **dadurch gekennzeichnet, dass** die chemisch inerte Matrix (8) aus der Gruppe ausgewählt ist, die Hydroxypropylmethylcellulose umfasst.

4. Ausrüstung nach Anspruch 1, **dadurch gekennzeichnet, dass** die mindestens eine ferromagnetische Zusammensetzung (7) aus der Gruppe ausgewählt ist, die Magnetit umfasst.

5. Ausrüstung nach Anspruch 1, **dadurch gekennzeichnet, dass** die Partikel der chemisch inerten Matrix (8) vor der Einkapselung oder Kompression mit der ferromagnetischen Zusammensetzung (7) gemischt werden, wobei die Partikel der inerten Matrix (8) nach Gewicht relativ zu den Partikeln der ferromagnetischen Zusammensetzung überwiegen(7).

6. Ausrüstung nach Anspruch 5, **dadurch gekennzeichnet, dass** die Menge an inerter Matrix (8) 70% Gewicht und die Menge an ferromagnetischer Zusammensetzung (7) 30% Gewicht beträgt.

7. Ausrüstung nach Anspruch 1, **dadurch gekennzeichnet, dass** die äußere Schicht (10) aus einem Material hergestellt ist, das aus der Gruppe ausgewählt ist, die Polymere mit freien funktionellen Gruppen wie Dextran, Polyethylenglykol (PEG), Polyethylenoxid (PEO), Gold, Aktivkohle und Kieselsäure umfasst.

8. Ausrüstung nach Anspruch 1, **dadurch gekennzeichnet, dass** die inerte Matrix (8) ferner Gleitmittel, Verdünner und Schmiermittel umfasst, die unter den Partikeln der ferromagnetischen Zusammensetzung (7) dispergiert sind, um ihr eine regelmäßigere Form zu verleihen und die Reibung zwischen diesen zu verringern.

9. Ausrüstung nach Anspruch 1, **dadurch gekennzeichnet, dass** das einnehmbare Element (2) in Kapselform (5) solche Abmessungen aufweist, dass es ein Volumen im Bereich von 0,7 ml bis 5 ml aufweist, und in Tablettenform (4) Abmessungen mit einem Durchmesser von 5 mm bis 20 mm, dies im Hinblick auf die Optimierung der Mitnahme und Entfernung von Fäkalomen (F).

10. Ausrüstung nach Anspruch 1, **dadurch gekennzeichnet, dass** sie bildbasierte Diagnosemittel (20) umfasst, die angepasst sind, um mit dem einnehmbaren Element (2) zu interagieren, um seine Position im Dickdarm (C) vor oder während der Bewegung durch die Magnetvorrichtung (3) zu erfassen, wobei das Diagnosemittel (20) aus der Gruppe ausgewählt ist, die einen Röntgenbildanalysator umfasst.

11. Ausrüstung nach einem oder mehreren der Ansprüche 1 bis 10 zur Verwendung in der Medizin.

12. Ausrüstung nach einem oder mehreren der Ansprüche 1 bis 10 zur Verwendung in der Veterinärmedizin.

## Revendications

1. Appareil (1) pour traiter la constipation et éliminer les fécalomes (F), comprenant:
- au moins un élément (2) destiné à être ingéré par un patient (P), qui se présente sous la forme d'un comprimé (4) ou d'une capsule (5) et présente des propriétés ferromagnétiques;
- un dispositif magnétique (3) adapté pour interagir avec ledit élément ingérable (2) à travers le corps (B) du patient (P);
dans lequel ledit dispositif magnétique (3) est conçu pour être placé à l'extérieur du patient (P), en correspondance de son côlon (C), des moyens de déplacement (12) étant associés audit dispositif magnétique (3) pour favoriser un mouvement contrôlé dudit élément ingérable (2) dans le côlon (C);
dans lequel ledit élément ingérable (2) comprend une couche externe (10) qui renferme un noyau (6) d'un matériau comprenant au moins un composé ferromagnétique (7) sous forme de poudre dispersée dans une matrice chimiquement inerte (8), ladite couche externe (10) étant biocompatible pour éviter une exposition directe du noyau (6) au corps (B) du patient (P);
**caractérisé en ce que** ledit dispositif magnétique (3) comprend en outre au moins un aimant permanent (13) associé à un support respectif (14) qui est conçu pour être tenu par un opérateur et qui définit lesdits moyens de déplacement (12), ledit dispositif magnétique (3) étant adapté pour être déplacé de façon répétée afin de provoquer la rupture mécanique des fécalomes (F) due aux chocs entre ces derniers et l'élément ingérable (2) et la suppression de l'obstruction au niveau du côlon (C), ladite couche externe (10) étant insoluble, non absorbable et capable de résister à l'environnement gastro-intestinal, de sorte que ledit élément ingérable (2) peut atteindre le côlon (C) du patient (P) sous une forme intacte, entrer en contact avec, casser et pousser les fécalomes (F) et faire avancer le contenu intestinal dans la direction aborale et être expulsé dans un état inchangé avec les matières fécales, l'appareil comprenant en outre un support (16) pour le corps (B) du patient (P) ledit dispositif magnétique (3) comprenant en outre au moins une paire d'électroaimants (15) placés sur des côtés opposés par rapport au patient (P), ledit moyen de mouvement (12) consistant en ledit support (16) pour communiquer le mouvement par rapport audit élément ingérable (2).

2. Appareil selon la revendication 1, **caractérisé en ce que** ledit noyau (6) est enfermé dans une couche de protection externe (9) qui est à son tour recouverte par et en contact direct avec ladite couche externe (10).

3. Appareil selon la revendication 1, **caractérisé en ce que** ladite matrice chimiquement inerte (8) est sélectionnée dans le groupe comprenant l'hydroxypropylméthylcellulose.

4. Appareil selon la revendication 1, **caractérisé en ce que** ledit au moins un composé ferromagnétique (7) est choisi dans le groupe comprenant la magnétite.

5. Appareil selon la revendication 1, **caractérisé en ce que** les particules de ladite matrice chimiquement inerte (8) sont mélangées au composé ferromagnétique (7) avant encapsulation ou compression, les particules de la matrice inerte (8) étant majoritairement en poids par rapport aux particules dudit composé ferromagnétique (7).

6. Appareil selon la revendication 5, **caractérisé en ce que** la quantité de matrice inerte (8) est de 70% en poids et la quantité de composé ferromagnétique (7) est de 30% en poids.

7. Appareil selon la revendication 1, **caractérisé en ce que** ladite couche externe (10) est en un matériau choisi dans le groupe comprenant des polymères à fonctions libres tels que le dextrane, le polyéthylène glycol (PEG), l'oxyde de polyéthylène (PEO), or, charbon actif et silice.

8. Appareil selon la revendication 1, **caractérisé en ce que** ladite matrice inerte (8) comprend en outre des agents de glissement, des diluants et des lubrifiants dispersés parmi les particules dudit composé ferromagnétique (7), pour lui conférer une forme plus régulière et réduire le frottement entre leur.

9. Appareil selon la revendication 1, **caractérisé en ce que** ledit élément ingérable (2) sous forme de capsule (5) a des dimensions telles qu'il a un volume allant de 0,7 ml à 5 ml, et sous forme de comprimé (4) a une telle dimension ayant un diamètre compris entre 5 mm et 20 mm, en vue d'optimiser l'entraînement et l'élimination des fécalomes (F).

10. Appareil selon la revendication 1, **caractérisé en ce qu'**il comprend des moyens de diagnostic à base d'image (20) qui sont adaptés pour interagir avec ledit élément ingérable (2) pour détecter sa position dans le côlon (C) avant ou pendant le mouvement par ledit dispositif magnétique (3), lesdits moyens de diagnostic (20) étant sélectionnés dans le groupe comprenant un analyseur d'images à rayons X.

11. Appareil selon l'une ou plusieurs des revendications 1 à 10, destiné à être utilisé en médecine.

12. Appareil selon l'une ou plusieurs des revendications 1 à 10, destiné à être utilisé en médecine vétérinaire.
